# FASCICULE DE BREVET EUROPEEN

(11) **EP 2 084 119 B1**
(45) Date de publication et mention de la délivrance du brevet: **11.04.2018**
(21) Numéro de dépôt: 07858541.1
(22) Date de dépôt: 10.10.2007
(51) Int. Cl.: C07C 17/21, C07C 19/08

(54) **PROCEDE DE FABRICATION DU PENTAFLUOROETHANE**
VERFAHREN ZUR HERSTELLUNG VON PENTAFLUORETHAN
PROCESS FOR THE MANUFACTURE OF PENTAFLUOROETHANE

(30) Priorité: 20.10.2006 FR 0654390
(43) Date de publication de la demande: 05.08.2009
(73) Titulaire: ARKEMA FRANCE, 92700 Colombes (FR)
(72) Inventeur: BOUSSAND, Béatrice, 69110 Sainte Foy Les Lyon (FR); GUIRAUD, Emmanuel, 69230 Saint-Genis-Laval (FR)
(74) Mandataire: Dang, Doris
(86) Numéro de dépôt international: PCT/FR2007/052111
(87) Numéro de publication internationale: WO 2008/050027

(56) Documents cités:
- EP-A1- 0 609 123
- WO-A-01/77048
- WO-A-95/32168
- US-A- 5 847 244

## Description

La présente invention concerne un procédé de fabrication du pentafluoroéthane. Elle a, plus particulièrement, pour objet un procédé continu de fabrication du pentafluoroéthane par fluoration du perchloroéthylène (PER) en phase gazeuse en présence d'un catalyseur.

Un des points critiques d'un procédé de fluoration en phase gazeuse est la stabilité du catalyseur.

Plusieurs solutions ont été suggérées pour maintenir la stabilité du catalyseur.

Ainsi le document EP 609123 décrit un procédé continu de fluoration catalytique du perchloroéthylène en phase gazeuse au moyen d'acide fluorhydrique en présence d'un catalyseur mixte composé d'oxydes, halogénures et/ou oxyhalogénures de nickel et de chrome déposés sur un support constitué de fluorure d'aluminium ou d'un mélange de fluorure d'aluminium et d'alumine.

La stabilité du catalyseur est mise en évidence dans l'exemple 2 de ce document avec une température de 350°C, à pression atmosphérique, un rapport molaire HF/PER voisin de 7 et un temps de contact de 15 secondes.

Lors de la fluoration du perchloroéthylène au moyen d'acide fluorhydrique en présence d'un catalyseur, il se forme un mélange de composés, avec majoritairement les composés de la « série F 120 », à savoir le F 121 (CHCl₂-CCl₂F), le F 122 (CHCl₂-CClF₂), le F 123 (CHCl₂-CF₃), le F 124 (CHFCl-CF₃), le F 125 (CHF₂-CF₃) ou leurs isomères. Outre les composés de la « série F120 », le mélange contient notamment du F 115 (CF₃-CF₂Cl), du F 114a (CF₃-CFCl₂),du F 114 (CF₂Cl-CF₂Cl), du F 133a (CH₂Cl-CF₃) et des oléfines F 1111 (CFCl=CCl₂) et F 1112a (CF₂=CCl₂).

Par ailleurs, la pression de la réaction de fluoration est souvent imposée par les étapes de séparation et purification, notamment la séparation d'HCl.

Ainsi, le document EP 734366 décrit un procédé de fabrication du pentafluoroéthane en faisant réagir dans la première étape un perhaloéthylène ou pentahaloéthane avec de l'HF en phase gaz en présence d'un catalyseur. Ce document enseigne de mettre en oeuvre cette étape à une pression pouvant aller jusqu'à 30 bar absolu pour faciliter la circulation du flux gazeux dans l'installation.

Le document EP 1110936 décrit une méthode de préparation des composés fluoroéthanes en faisant réagir au moins un composé choisi parmi le PER, le dichlorotrifluoroéthane (123) et 124 avec de l'HF en présence d'un catalyseur d'oxyfluorure de chrome ayant une teneur en fluor d'au moins 30 % en poids. Ce document enseigne d'opérer la réaction de fluoration à une pression qui sera fonction des conditions de séparation des produits et de purification.

Il a maintenant été trouvé un procédé de fabrication du pentafluoroéthane en faisant réagir le perchloroéthylène avec de l'HF en phase gazeuse en présence d'un catalyseur et ne présentant pas les inconvénients précités.

L'invention a donc pour objet un procédé de fabrication du pentafluoroéthane à partir du perchloroéthylène et d'HF en phase gazeuse en présence d'un catalyseur caractérisé en ce que (i) la réaction du PER avec de l'HF est mise en oeuvre avec un ratio molaire HF/PER supérieur à 20 et une pression supérieure à 5 bar absolu et à une température comprise entre 330°C et 375°C, en présence d'oxygène et d'un catalyseur mixte composé d 'oxydes, d'halogénures et/ou d'oxyhalogénures de nickel et de chrome déposés sur un support constitué de fluorure d'aluminium ou d'un mélange de fluorure d'aluminium et d' alumine, et que (ii) le flux sortant de cette étape réactionnelle est soumis à une étape de séparation pour donner une fraction (A) de produits légers comprenant de l'acide chlorhydrique, du pentafluoroéthane et une fraction (B) de produits lourds comprenant de l'acide fluorhydrique non réagi, éventuellement du perchloroéthylène non réagi et que (iii) l'on recycle directement à l'étape réactionnelle la fraction (B), sans aucune opération de purification.

La fraction B peut comprendre en outre le dichlorotrifluoroéthane et/ou chlorotetrafluoroéthane formé dans l'étape réactionnelle.

Le ratio molaire HF/PER dans l'étape réactionnelle est de préférence compris entre 20 et 60, et avantageusement compris entre 25 et 50.

L'étape réactionnelle est de préférence mise en oeuvre à une pression comprise entre 6 et 15 bar absolu et avantageusement comprise entre 6 et 12 bar absolu.

Dans le procédé selon la présente invention le temps de contact divisé par la pression absolue est compris entre 2 et 6 s/ bar, de préférence entre 2,5 et 4 s/bar. Le temps de contact est calculé comme étant le temps de passage des gaz dans les conditions réactionnelles à travers le volume de catalyseur.

Pour cette invention, on utilise des catalyseurs mixtes composés d'oxydes, d'halogénures et/ou d'oxyhalogénures de nickel et de chrome déposés sur un support constitué de fluorure d'aluminium ou d'un mélange de fluorure d'aluminium et d'alumine tels que décrits par exemple dans les brevets FR 2 669 022 et EP-B-0 609 124.

Lorsqu'on utilise un catalyseur mixte de nickel/chrome, on recommandera les catalyseurs contenant, en masse, de 0,5 à 20 % de chrome et de 0,5 à 20 % de nickel et plus particulièrement ceux contenant de 2 à 10 % en masse de chacun des métaux dans un rapport atomique nickel/chrome compris entre 0,1 et 5, de préférence voisin de 1. La teneur d'oxygène à utiliser dans la réaction de fluoration peut varier selon les conditions opératoires entre 0,02 et 2 % molaire par rapport aux réactifs entrant dans le réacteur. L'introduction de l'oxygène pourra se faire de manière continue ou séquentielle.

Le procédé selon la présente invention peut être mis en oeuvre aussi bien en continu qu'en discontinu mais, on préfère opérer en continu.

### PARTIE EXPERIMENTALE

Le catalyseur utilisé est un catalyseur mixte Nickel/Chrome de rapport atomique Ni/ Cr = 1, supporté sur de l'alumine préalablement fluorée et est préparé par imprégnation de solutions de sel de nickel et anhydride chromique (CrO₃).

Après imprégnation et séchage, le solide est soumis à un traitement à une température comprise entre 320 et 390°C, en présence d'un mélange d'acide fluorhydrique et d'azote (concentration volumique de 5 à 10 % de cet acide dans l'azote).

Les exemples ont été réalisés à l'aide d'un pilote de fluoration, constitué d'un évaporateur, d'un réacteur de fluoration (101), d'une colonne à distiller (102) et d'une boucle de recyclage (107). On alimente les réactifs, en phase gazeuse, en continu (perchloroéthylène (104) et acide fluorhydrique (103) après les avoir évaporés dans (108) et (109)) dans un réacteur de fluoration (101) en Inconel contenant 48,8 I de catalyseur. En sortie du réacteur (105), une colonne à distiller (102) permet de séparer les produits de la réaction, tels que F 125, HCl, HF (entraîné sous forme d'azéotrope avec les organiques) et tout ou partie de F 123 et F 124 d'une part (106), des réactifs non transformés et produits sous-fluorés (PER, HF en excès et tout ou partie de F 123, F 124) d'autre part (107).

Le pied de colonne (107) comprenant les réactifs non transformés et les produits sous-fluorés est recyclé au réacteur après les avoir évaporés dans (110).

On maintient un niveau constant dans le bouilleur de la colonne à distiller grâce au débit de recyclage et débit des réactifs frais.

On introduit de l'air (111) dans le réacteur en quantité telle qu'elle correspond au ratio molaire indiqué ci-dessous.

Les conditions opératoires dans le réacteur de fluoration du PER sont les suivantes :
- Température du four régulée à 350°C
- Pression : 7 bar abs
- Ratio Molaire HF / PER = 41
- Ratio Molaire O₂/(HF + organiques) = 0,1 %

Temps de contact = 23,3 s (donc temps de contact/Pabs = 3,3 s/bar) Après 500 h de marche, la productivité en F 125 récupéré en tête de la colonne à distiller est restée stable et égale à 50 g/h/l de catalyseur et la conversion du PER est totale en sortie de réacteur.

La composition (%mol) des trois flux principaux est stable :

| | Sortie réacteur (105) | Tête de colonne (106) | Recyclage (107) |
|---|---|---|---|
| F125 | 2,0 | 17,8 | - |
| F124/F124a | 2,4 | 1,8 | 2,5 |
| F 123/F123a | 0,6 | - | 0,6 |
| F122/F122a | 0,01 | - | < 0,01 |
| autres | 0,6 | 4,7 | 0,1 |
| HF | 86,2 | 3,8 | 96,8 |
| HCl | 8,2 | 71,9 | - |

### Exemple comparatif :

Volume de catalyseur : 17.8 litres
Température du four régulée à 310°C
   - Pression : 7 bar abs
   - Ratio Molaire HF / PER = 7
   - Ratio Molaire O₂/ (HF + organiques) = 0.1% Temps de contact = 40 s (donc temps de contact/Pabs = 5,7 s/bar).

Les résultats sont présentés dans le tableau ci-dessous :

| Durée (h) | Conversion du PER en sortie de réacteur (%) | Production de F123+F124+F125 (g/h/l) | Production de F125 (g/h/l) |
|---|---|---|---|
| 75 | 96,9 | 192 | 7,8 |
| 198 | 94,3 | 169 | 4,1 |
| 325 | 90,8 | 160 | 2,9 |
| 405 | 88,6 | 148 | 2 |
| 682 | 81,5 | 100 | 0,74 |
| 819 | 72,5 | 83 | 0,7 |

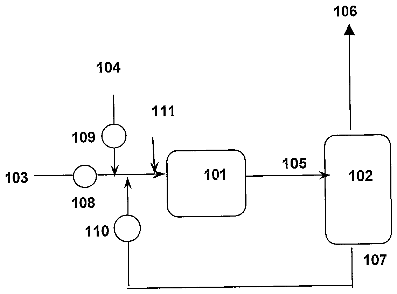

## Revendications

1. Procédé de fabrication du pentafluoroéthane à partir du perchloroéthylène et d'HF en phase gazeuse en présence d'un catalyseur **caractérisé en ce que** (i) la réaction du PER avec de l'HF est mise en oeuvre avec un ratio molaire HF/PER supérieur à 20, une pression supérieure à 5 bar absolu à une température comprise entre 330°C et 375°C, en présence d'oxygène et d'un catalyseur mixte composé d'oxydes, d'halogénures et/ou d'oxyhalogénures de nickel et de chrome déposés sur un support constitué de fluorure d'aluminium ou d'un mélange de fluorure d'aluminium et d'alumine, et que (ii) le flux sortant de cette étape réactionnelle est soumis à une étape de séparation pour donner une fraction (A) de produits légers comprenant de l'acide chlorhydrique, du pentafluoroéthane et une fraction (B) de produits lourds comprenant de l'acide fluorhydrique non réagi, éventuellement du perchloroéthylène non réagi et que (iii) l'on recycle directement à l'étape réactionnelle la fraction (B), sans aucune opération de purification.

2. Procédé selon la revendication 1 **caractérisé en ce que** la fraction B peut comprendre en outre le dichlorotrifluoroéthane et/ou chlorotetrafluoroéthane formé dans l'étape réactionnelle.

3. Procédé selon la revendication 1 ou 2 **caractérisé en ce que** le ratio molaire HF/PER dans l'étape réactionnelle est compris entre 20 et 60.

4. Procédé selon l'une quelconque des revendications précédentes **caractérisé en ce que** l'étape réactionnelle est mise en oeuvre à une pression comprise entre 6 et 15 bar absolu.

5. Procédé selon l'une quelconque des revendications précédentes **caractérisé en ce que** le temps de contact divisé par la pression est compris entre 2 et 6 s/bar.

## Patentansprüche

1. Verfahren zur Herstellung von Pentafluorethan aus Perchlorethylen und HF in der Gasphase in Gegenwart eines Katalysators, **dadurch gekennzeichnet, dass** (i) die Reaktion von PER mit HF mit einem HF/PER-Molverhältnis von mehr als 20 bei einem Druck von mehr als 5 bar absolut und einer Temperatur zwischen 330 °C und 375 °C in Gegenwart von Sauerstoff und einem Mischkatalysator aus Oxiden, Halogeniden und/oder Oxidhalogeniden von Nickel und Chrom, die auf einem Träger aus Aluminiumfluorid oder einer Mischung von Aluminiumfluorid und Aluminiumoxid abgeschieden sind, durchgeführt wird, und dass (ii) der aus diesem Reaktionsschritt austretende Strom einem Trennschritt unterworfen wird, der eine Fraktion (A) von leichten Produkten, die Salzsäure und Pentafluorethan umfasst, und eine Fraktion (B) von schweren Produkten, die nicht umgesetzte Fluorwasserstoffsäure und gegebenenfalls nicht umgesetztes Perchlorethylen umfasst, ergibt, und dass (iii) die Fraktion (B) ohne jeglichen Reinigungarbeitsgang direkt in den Reaktionsschritt zurückgeführt wird.

2. Verfahren nach Anspruch 1, **dadurch gekennzeichnet, dass** die Fraktion B zusätzlich im Reaktionsschritt gebildetes Dichlortrifluorethan und/oder Chlortetrafluorethan umfassen kann.

3. Verfahren nach Anspruch 1 oder 2, **dadurch gekennzeichnet, dass** das HF/PER-Molverhältnis im Reaktionsschritt zwischen 20 und 60 liegt.

4. Verfahren nach einem der vorhergehenden Ansprüche, **dadurch gekennzeichnet, dass** der Reaktionsschritt bei einem Druck zwischen 6 und 15 bar absolut durchgeführt wird.

5. Verfahren nach einem der vorhergehenden Ansprüche, **dadurch gekennzeichnet, dass** die Kontaktzeit dividiert durch den Druck zwischen 2 und 6 s/bar liegt.

## Claims

1. Process for the manufacture of pentafluoroethane from perchloroethylene and HF in the gas phase in the presence of a catalyst, **characterized in that** (i) the reaction of PER with HF is carried out with an HF/PER molar ratio of greater than 20, a pressure greater than 5 bar absolute, at a temperature of between 330°C and 375°C, in the presence of oxygen and of a mixed catalyst composed of oxides, of halides and/or of oxyhalides of nickel and of chromium deposited on a support composed of aluminum fluoride or of a mixture of aluminum fluoride and of alumina, and that (ii) the stream exiting from this reaction stage is subjected to a separation stage in order to give a fraction (A) of light products comprising hydrochloric acid and pentafluoroethane and a fraction (B) of heavy products comprising unreacted hydrofluoric acid and possibly unreacted perchloroethylene and that (iii) the fraction (B) is recycled directly to the reaction stage without any purification operation.

2. Process according to Claim 1, **characterized in that** fraction B can additionally comprise dichlorotrifluoro-ethane and/or chlorotetrafluoroethane formed in the reaction stage.

3. Process according to Claim 1 or 2, **characterized in that** the HF/PER molar ratio in the reaction stage is between 20 and 60.

4. Process according to any one of the preceding claims, **characterized in that** the reaction stage is carried out at a pressure of between 6 and 15 bar absolute.

5. Process according to any one of the preceding claims, **characterized in that** the contact time divided by the pressure is between 2 and 6s/bar.
